# EUROPÄISCHE PATENTANMELDUNG

(11) **EP 0 976 730 A1**
(43) Veröffentlichungstag der Anmeldung: **02.02.2000**
(21) Anmeldenummer: 99114111.0
(22) Anmeldetag: 16.07.1999
(51) Int. Cl.: C07D 207/44

(54) **Verfahren zur Herstellung von aromatischen Maleinimiden**

(30) Priorität: 28.07.1998 DE 19833912
(71) Anmelder: RHEIN-CHEMIE RHEINAU GmbH, 68219 Mannheim (DE)
(72) Erfinder: Heiliger, Ludger, Dr., 67433 Neustadt/Weinstrasse (DE); Herpich, Rüdiger, Dr., 68163 Mannheim (DE); Käsbauer, Josef, Dr., 42929 Wermelskirchen (DE); Ullrich, Friedrich-Wilhelm, Dr., 51465 Bergisch Gladbach (DE)
(74) Vertreter: Feldhues, Michael L.F., Dr.

(57) **Zusammenfassung**

Aromatische Maleinimide können hergestellt werden, indem man entsprechende aromatische Amine mit Maleinsäureanhydrid in Gegenwart eines Lösungsmittelgemisches aus halogeniertem Kohlenwasserstoff und einem dipolaren aprotischen Lösungsmittel unter Zugabe von Säure und eines Polymerisationsinhibitors umsetzt.

## Beschreibung

Die Erfindung betrifft ein verbessertes Verfahren zur Herstellung von aromatischen Maleinimiden durch Umsetzung von entsprechenden aromatischen Aminen mit Maleinsäureanhydrid.

Die Herstellung von aromatischen Maleinimiden ist bekannt. So wird beispielsweise in DE-A 2 127 025 ein Verfahren zur Herstellung von aromatischen Maleinimiden beschrieben, das in zwei Stufen abläuft. In der ersten Stufe erfolgt die Addition der Aromaten an Maleinsäureanhydrid in Tetrahydrofuran als Lösungsmittel. In der zweiten Stufe findet unter Wasserabspaltung eine Ringschlußreaktion des Maleinsäuremonoamids zu Maleinimid statt. Hierbei müssen auf die Amidgruppen bezogen äquimolare Mengen an Acetanhydrid und Natriumacetat eingesetzt werden. Gemäß DE-A 2 715 503 wird die Ringschlußreaktion statt mit Natriumacetat mit Triethylamin durchgeführt und gemäß DE-A 2 719 903 kommt für die Ringschlußreaktion ein Mangan-/Kobalt-/Lithiumsalz zum Einsatz.

Nachteilig bei den oben beschriebenen Verfahren zur Herstellung von aromatischen Maleinimiden ist, daß beispielsweise Tetrahydrofuran als Lösungsmittel eingesetzt wird, das wegen seiner Neigung zur Peroxidbildung und seines niedrigen Flammpunktes einen erheblichen Aufwand an entsprechenden Sicherheitsmaßnahmen erfordert. Ähnlich verhält sich die Situation beim Einsatz von Triethylamin, das einen vergleichbar niedrigen Flammpunkt wie Tetrahydrofuran besitzt. Der Einsatz von Acetanhydrid erfolgt stöchiometrisch und liefert nach der Reaktion äquimolare Mengen an Essigsäure, die entsprechend entsorgt werden müssen.

Das nach der Umsetzung anfallende Lösungsmittelgemisch aus beispielsweise Tetrahydrofuran, Essigsäure, gegebenenfalls Resten an Essigsäureanhydrid und Triethylamin gekoppelt mit Produktresten und Nebenprodukten, kann nicht ohne größeren technischen Aufwand aufgearbeitet werden oder der Wiederverwendung ohne entsprechende Reinigung zugeführt werden.

Aufgabe der vorliegenden Erfindung war es nun, ein ökonomisch und ökologisch vorteilhaftes Verfahren zur Herstellung von aromatischen Maleinimiden zur Verfügung zu stellen, das die obengenannten Nachteile vermeidet und eine entsprechend höhere Wirtschaftlichkeit und Umweltverträglichkeit mit sich bringt. Die Verwendung von explosionsgefährlichen Lösungsmitteln soll bei dem erfindungsgemäßen Verfahren vermieden werden und es sollen keine stöchiometriseh gebildeten Nebenprodukte anfallen, die entsprechend entsorgt werden müssen.

Gegenstand der vorliegenden Erfindung ist daher ein Verfahren zur Herstellung von aromatischen Maleinimiden der Formeln (I) bis (IV) 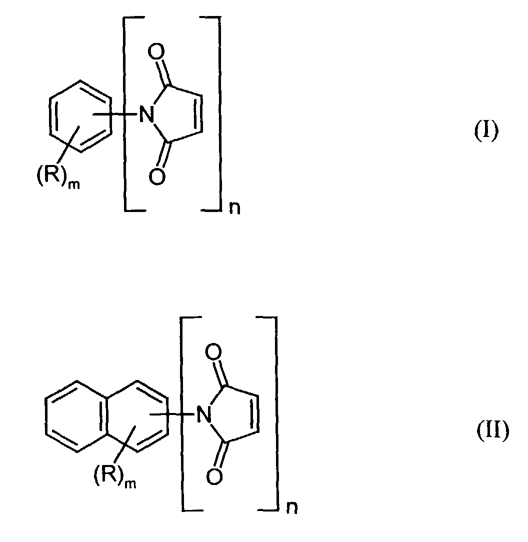 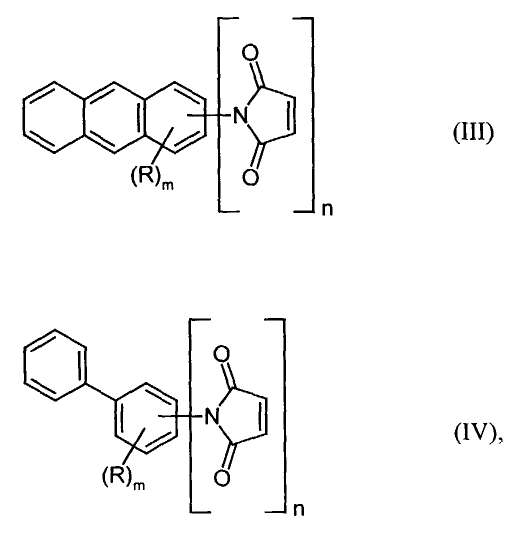 worin
R für C₁-C₁₈-Alkylreste steht,
n eine ganze Zahl von 2 bis 4 bedeutet und
m für ganze Zahlen von 0 bis 3 steht,
   das dadurch gekennzeichnet ist, daß man aromatische Amine der Formeln (V) bis (VIII) 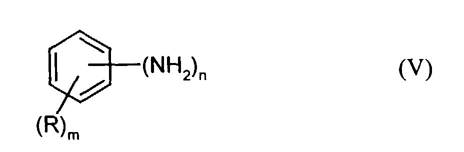 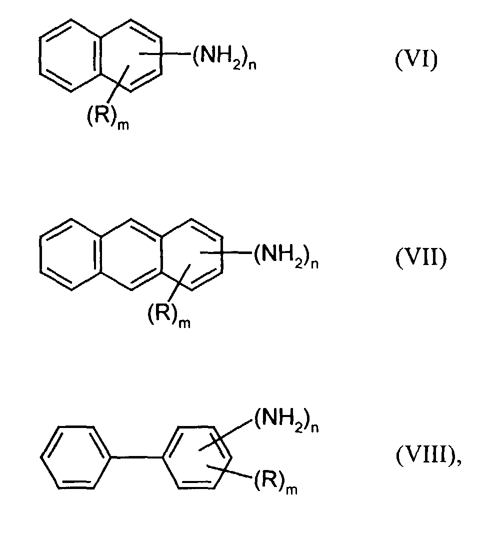 worin
R, n und m die zuvor genannten Bedeutungen besitzen,
   mit Maleinsäureanhydrid in Gegenwart eines Lösungsmittelgemisches aus
   a) 50 bis 99 Gew.-% eines gegebenenfalls halogenierten Kohlenwasserstoffs und
   b) 1 bis 50 Gew.-% eines dipolaren aprotischen Lösungsmittels
   unter Zugabe von 0,1 bis 10 Gew.-%, bezogen auf Maleinsäureanhydrid, einer Säure und 0,01 bis 2 Gew.-%, bezogen auf Maleinsäureanhydrid, eines Polymerisationsinhibitors umsetzt.

In den zuvor genannten Formeln steht R bevorzugt für C₁- bis C₁₂-, insbesondere C₁- bis C₄-Alkylreste, n bevorzugt für 2-3 und m bevorzugt für 1-2.

Bevorzugt werden als Lösungsmittelgemische solche eingesetzt, die aus
a) 70 bis 99 Gew.-% eines gegebenenfalls halogenierten Kohlenwasserstoffs und
b) 1 bis 30 Gew.-% eines dipolaren aprotischen Lösungsmittels bestehen.

In der bevorzugten Ausgestaltung des erfindungsgemäßen Verfahrens wird unter Zugabe von 0,3 bis 8 Gew.-% einer Säure und 0,1 bis 1 Gew.-% eines Polymerisationsinhibitors gearbeitet.

Als aromatische Amine (V) bis (VIII), die nach erfindungsgemäßen Verfahren eingesetzt werden können, werden beispielsweise genannt: Phenylendiamine, Toluylendiamine, Naphthylamine, Diaminonaphthaline, Aminoanthracene, Benzidin, Diaminobenzidine, vorzugsweise 2,4-Toluylendiamin und 1,3-Phenylendiamin. Die vorgenannten aromatischen Amine können sowohl einzeln als auch im Gemisch untereinander eingesetzt werden.

Als gegebenenfalls halogenierte Kohlenwasserstoffe können entsprechende aliphatische, cycloaliphatische und aromatische Kohlenwasserstoffe eingesetzt werden, die ein- oder mehrfach durch Halogenatome, wie Fluor, Chlor oder Brom, substituiert sind und die bei Raumtemperatur in flüssiger Form vorliegen. Beispielsweise eignen sich für das erfindungsgemäße Verfahren Toluol, Xylol, Cyclohexan, Isooctan und Chlorbenzol, bevorzugt Toluol, Xylol und Chlorbenzol. Die erfindungsgemäß einzusetzenden Kohlenwasserstoffe können sowohl einzeln als auch im Gemisch untereinander eingesetzt werden.

Als geeignete dipolare aprotische Lösungsmittel für das erfindungsgemäße Verfahren sind z.B. zu nennen: N-Methylpyrrolidon, Dimethylsulfoxid, Dimethylacetamid, Dimethylformamid, Formamid, Ethylen- und Propylenglycole sowie Dialkylcarbonate, bevorzugt N-Methylpyrrolidon, Dimethylformamid und Ethylen- und Propylenglycol, besonders bevorzugt sind N-Methylpyrrolidon, Dimethylformamid und Dimethylacetamid. Auch diese Lösungsmittel können sowohl einzeln als auch im Gemisch untereinander eingesetzt werden.

Als Säure kommen in z.B. Frage: Mineralsäuren, wie Salzsäure, Schwefelsäure, Phosphorsäure, sowie Lewis-Säure, wie Bortrifluorid, Bortrichlorid, Eisentrichlorid, Zinkdichlorid, Dialkylzinndialkanoate, Säureanhydride, wie Diphosphorpentoxid und Trifluormethansulfonsäureanhydrid, saure Ionenaustauscher auf der Basis sulfonierter Styrol-Divinylbenzol-Harze (z.B. Levatit®-Harze) sowie starke organische Säuren, wie para-Toluolsulfonsäure, Trifluormethansulfonsäure und Trifluoressigsäure. Bevorzugt sind Salzsäure, Phosphorsäure, Diphosphorpentoxid, para-Toluolsulfonsäure, Trifluormethansulfonsäure und Trifluoressigsäure. Ganz besonders bevorzugt sind para-Toluolsulfonsäure, Phosphorsäure und Diphosphorpentoxid.

Als Polymerisationsinhibitoren sind vor allem zu nennen alle (mehrkernigen) Phenole, bevorzugt sind Hydrochinon, para-Methoxyphenol, 2,6-Di-tert.-butyl-para-methylphenol, besonders bevorzugt sind Hydrochinon und para-Methoxyphenol.

Die genannten Säuren und Polymerisationsinhibitoren können ebenfalls sowohl einzeln als auch im Gemisch untereinander eingesetzt werden, wobei das günstigste Mischungsverhältnis jeweils durch entsprechende Vorversuche ermittelt werden kann.

Nach dem erfindungsgemäßen Verfahren werden die aromatischen Amine in dem Lösungsmittelgemisch in Anwesenheit des Polymerisationsinhibitors mit Maleinsäureanhydrid bei Temperaturen von 0 bis 80°C, vorzugsweise bei Temperaturen von 20 bis 70°C, besonders bevorzugt bei 40 bis 60°C, zur Reaktion gebracht und solange gerührt, bis kein freies Amin mehr nachzuweisen ist. Der Nachweis kann mit den üblichen Methoden der Reaktionsverfolgung erbracht werden, wie durch Gaschromatographie oder Dünnschichtchromatographie. Wenn kein freies Amin mehr nachzuweisen ist, erfolgt die Säurezugabe. Die Reaktionsmischung wird dann auf 90 bis 140°C, vorzugsweise auf 100 bis 120°C, besonders bevorzugt auf 105 bis 115°C, erhitzt. Die Heizphase dauert so lange, bis die Wasserabscheidung unter Rückführung des Lösungsmittelgemisches beendet ist.

Die Umsetzung nach dem erfindungsgemäßen Verfahren kann auch in zwei Stufen erfolgen, wobei im ersten Schritt bei den angegebenen Temperaturen die aromatischen Amine im Lösungsmittelgemisch in Anwesenheit des Polymerisationsinhibitors mit Maleinsäureanhydrid über einen Zeitraum von bis zu 8 Stunden, bevorzugt 6 bis 8 Stunden, zur Reaktion gebracht werden. In der zweiten Stufe wird die Reaktionsmischung der ersten Stufe in eine Lösung der Katalysatorsäure im Lösungsmittelgemisch bei den angegebenen Temperaturen über einen Zeitraum von bis zu 6 Stunden zudosiert. Die Reaktionsmischung wird bei der oben angegebenen Temperatur noch maximal 6 Stunden lang gerührt, bevorzugt 4 - 6 Stunden bis die Wasserabscheidung beendet ist.

Das Produkt wird erfindungsgemäß nach erfolgter Umsetzung in der Weise isoliert, daß man nach dem Abkühlen auf Reaktionstemperatur den entstandenen Niederschlag abfiltriert, den Niederschlag wäscht, trocknet und umkristallisiert. Aus der Mutterlauge kann durch Abdestillieren des Lösungsmittels und Zusatz von Wasser ein weiterer Anteil am entsprechenden aromatischen Maleinimid gewonnen werden.

Die nachfolgenden Beispiele sollen das erfindungsgemäße Verfahren verdeutlichen.

### Beispiele

### Beispiel 1

### Synthese von 2,4-Bis(maleinimido)toluol

Man erwärmt ein Gemisch aus 523 g Toluol, 15 g N-Methylpyrrolidon, 0,3 g Hydrochinon und 98,1 g/1,0 mol Maleinsäureanhydrid unter Rühren auf 60°C und dosiert über einen Zeitraum von 3 Stunden 62 g/0,5 mol 2,4-Toluylendiamin hinzu. Man rührt 3 Stunden nach, fügt 7,0 g para-Toluolsulfonsäure hinzu und bringt den Kolbeninhalt unter Rühren bei 110°C zum Kochen. Nach 8,25 Stunden ist die azeotrope Wasserabscheidung beendet, man gießt die Lösung durch einen Faltenfilter und läßt zum Auskristallisieren abkühlen.

Man nutscht den Niederschlag ab, trocknet ihn bei 70°C im Vakuum und zerkleinert ihn.
Ausbeute: 100 g (entspricht 71 % der Theorie). Die Charakterisierung der Substanz erfolgte durch Elementaranalyse, TLC, IR und den Schmelzpunkt.
Schmelzpunkt: 160-164°C

| Elementaranalyse: C₁₅H₁₀N₂O₄ (282,3 g/mol) | | | |
|---|---|---|---|
| Ber.: | C: 63,8 | H: 3,6 | N: 9,9 |
| Gef.: | C: 64,2 | H: 3,9 | N: 9,9 |

TLC (Laufmittel: Ethanol, Trägermaterial: Kieselgel): R_{f}-Wert: 0,69
- IR:: 1700 cm-¹ Streckschwingungen C=O
3100 cm-¹ Streckschwingungen C_{(arom.)} - H
3500 cm-¹ Streckschwingungen C_{(aliphat.)} - H

### Beispiel 2

### Synthese von 1,3-Phenylenbis(maleinimid)

Man erwärmt ein Gemisch aus 196 g Toluol, 26 g N-Methylpyrrolidon, 0,15 g Hydrochinon und 24,5 g/0,25 mol Maleinsäureanhydrid unter Rühren auf 60°C und dosiert über einen Zeitraum von 1 Stunde 13,5 g/0,125 mol meta-Phenylendiamin hinzu. Man rührt 2 Stunden bei dieser Temperatur nach.

Diese Suspension tropft man im Zeitraum von 6 Stunden unter Rühren in eine siedende Lösung von 0,9 g para-Toluolsulfonsäure in 15,5 g N-Methylpyrrolidon und 118 g Toluol. Das entstehende Reaktionswasser wird unter Rückführung des Lösungsmittelgemischs azeotrop abdestilliert. Man rührt noch 2 Stunden nach und filtriert. Nach dem Abkühlen kann 1,3-Phenylenbis(maleinimid) abgenutscht, getrocknet und pulverisiert werden.

Aus der Mutterlauge gewinnt man einen weiteren Produktanteil durch Abdestillieren des Toluolanteils vom Lösemittelgemisch, Verrühren des Rückstandes mit 200 g Wasser, Abnutschen des Niederschlages und Trocknen bzw. Pulverisieren des Feststoffs.

Zum Reinigen kocht man das Produkt in Toluol, trennt in der Siedehitze durch Filtration Nebenprodukte ab und destilliert das Lösungsmittel ab.
Ausbeute: 24,5 g (entspricht 73 % der Theorie). Die Charakterisierung der Substanz erfolgte durch Elementaranalyse, TLC, IR und den Schmelzpunkt.
Schmelzpunkt: 198-199°C

| Elementaranalyse: C₁₄H₈N₂O₄ (268,2 g/mol) | | | |
|---|---|---|---|
| Ber.: | C: 62,7 | H: 3,0 | N: 10,4 |
| Gef.: | C: 62,3 | H: 3,2 | N: 10,5 |

TLC (Laufmittel: Ethanol, Trägermaterial: Kieselgel): R_{f}-Wert: 0,71
- IR:: 1600 cm-¹ Streckschwingungen olef. C=C
1720 cm-¹ Streckschwingungen C=O
2860 cm-¹ Streckschwingungen C_{(olef.)} - H
3100 cm-¹ Streckschwingungen C_{(arom.)} - H
3500 cm-¹ Streckschwingungen C_{(aliphat.)} - H

## Patentansprüche

1. Verfahren zur Herstellung von aromatische Maleinimiden der Formeln (I) bis (IV) 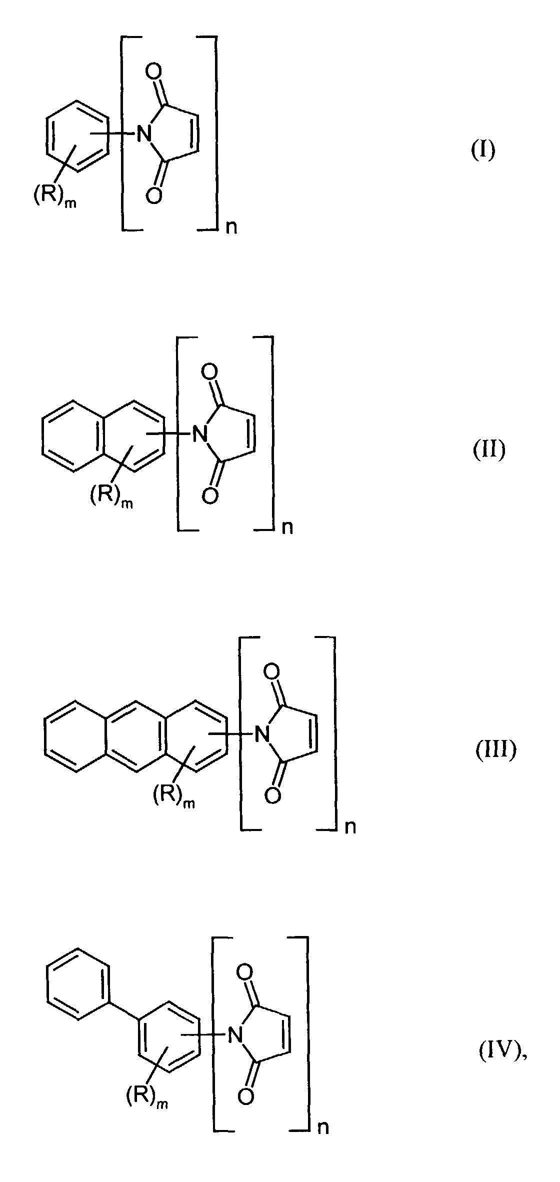 worin
R für C₁-C₁₈-Alkylreste steht,
n eine ganze Zahle von 2 bis 4 bedeutet und
m für ganze Zahlen von 0 bis 3 steht,
dadurch gekennzeichnet, daß man aromatische Amine der Formeln (V) bis (VIII) 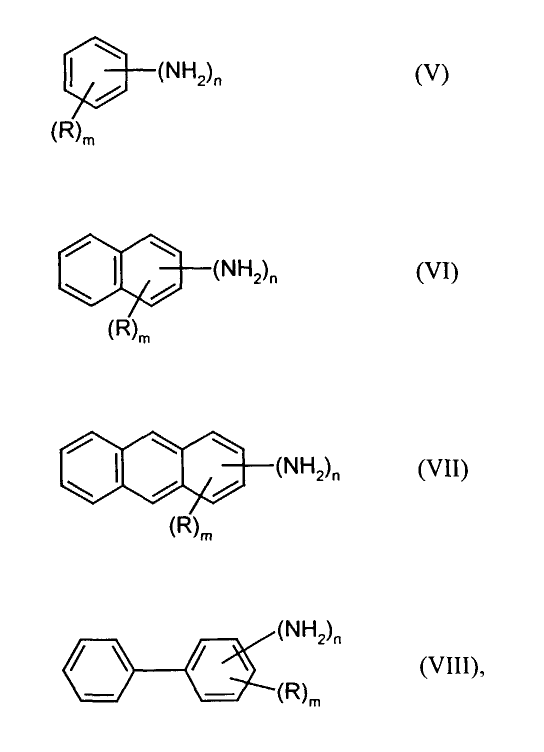 worin
R, n und m die zuvor genannten Bedeutungen besitzen,
mit Maleinsäureanhydrid in Gegenwart eines Lösungsmittelgemisches aus
a) 50 bis 99 Gew.-% eines gegebenenfalls halogenierten Kohlenwasserstoffs und
b) 1 bis 50 Gew.-% eines dipolaren aprotischen Lösungsmittels
unter Zugabe von 0,1 bis 10 Gew.-%, bezogen auf Maleinsäureanhydrid, einer Säure und 0,01 bis 2 Gew.-%, bezogen auf Maleinsäureanhydrid, eines Polymerisationsinhibitors umsetzt.

2. Verfahren nach Anspruch 1, dadurch gekennzeichnet, daß das Lösungsmittelgemisch aus 70 bis 99 Gew.-% eines gegebenenfalls halogenierten Kohlenwasserstoffs und 1 bis 30 Gew.-% eines dipolaren aprotischen Lösungsmittels besteht.

3. Verfahren nach Anspruch 1, dadurch gekennzeichnet, daß die Umsetzung unter Zugabe von 0,3 bis 8 Gew.-% einer Säure und 0,1 bis 1 Gew.-% eines Polymerisationsinhibitors, jeweils bezogen auf eingesetztes Maleinsäureanhydrid, erfolgt.
